# EUROPEAN PATENT SPECIFICATION

(11) **EP 3 094 369 B1**
(45) Date of publication and mention of the grant of the patent: **10.01.2018**
(21) Application number: 15701907.6
(22) Date of filing: 14.01.2015
(51) Int. Cl.: A61N 1/36

(54) **SYSTEMS FOR SELECTIVE STIMULATION OF NERVE FIBERS IN CAROTID SINUS**
SYSTEME ZUR SELEKTIVEN STIMULATION VON NERVENFASERN IM SINUS CAROTICUS
SYSTÈMES DE STIMULATION SÉLECTIVE DE FIBRES NERVEUSES DANS LE SINUS CAROTIDIEN

(30) Priority: 17.01.2014 US 201461928707 P
(43) Date of publication of application: 23.11.2016
(73) Proprietor: Cardiac Pacemakers, Inc., St. Paul, MN 55112-5798 (US)
(72) Inventor: FRANKE, Manfred, 09600 Weissenborn (SA) (DE); SHUROS, Allan C., St. Paul, Minnesota 55116 (US); MOKELKE, Eric A., Flagstaff, Arizona 86005 (US); HINCAPIE ORDONEZ, Juan Gabriel, Maple Grove, Minnesota 55369 (US); TERNES, David J., Roseville, Minnesota 55113 (US)
(74) Representative: Pfenning, Meinig & Partner mbB
(86) International application number: PCT/US2015/011458
(87) International publication number: WO 2015/109015

(56) References cited:
- WO-A1-2010/019481
- WO-A2-2013/018083
- US-A1- 2014 018 788

## Description

### CLAIM OF PRIORITY

This application claims the benefit of priority under 35 U.S.C. § 119(e) of U.S. Provisional Patent Application Serial No. 61/928,707, filed on January 17, 2014, which is herein incorporated by reference in its entirety.

### CROSS REFERENCE TO RELATED APPLICATIONS

The following commonly-assigned U.S. patent application are related, are all filed on the same date as the present application, and are all herein incorporated by reference in their entirety: "Systems and Methods for Delivering Pulmonary Therapy," Ser. No. 61/928,714, filed on January 17, 2014; "Depletion Block to Block Nerve Communication," Ser. No. 61/928,725, filed on January 17, 2014; and "Selective Nerve Stimulation Using Presynaptic Terminal Depletion Block," Ser. No. 61/928,732, filed on January 17, 2014.

### TECHNICAL FIELD

This document relates generally to medical devices, and more particularly, to systems and methods for selectively blocking or stimulating nerve fibers innervating the carotid sinus region.

### BACKGROUND

Therapies applied to neural tissue may include, but are not limited to, electrical stimulation to enhance nerve activity, electrical stimulation to reduce or block nerve activity, and ablation of neural pathways. For example, autonomic modulation therapy (AMT) includes therapies for respiratory problems such as sleep disordered breathing, blood pressure control such as to treat hypertension, cardiac rhythm management, myocardial infarction and ischemia, heart failure (HF), and modulation of the cholinergic antiinflammatory pathway. Therapies to treat epilepsy, depression, pain, migraines, eating disorders and obesity, and movement disorders may include stimulation of a vagus nerve.

It has been proposed to reduce blood pressure by electrically stimulating baroreceptor regions to induce a baroreflex response. Baroreceptors play an important role in regulating blood pressure, and are located throughout the body, but primarily in the arch of the aorta and the carotid sinuses of the left and right internal carotid arteries. Through a negative feedback baroreflex system, the central nervous system can regulate the blood pressure to maintain the blood pressure at a relatively stable level For example, arterial pressure that causes stretch triggers the baroreflex to send nerve impulses to the brain which responds by controlling the pumping activity of the heart and blood vessel dilation to reduce the blood pressure. The carotid body is a small cluster of cells located near the bifurcation of the carotid artery. The carotid body includes chemoreceptors that detect blood composition changes such as changes in partial pressure of oxygen and carbon dioxide. To treat breathing disorders, the carotid body has been removed, ablated or otherwise destroyed since the 1940s. More recently, the removal or ablation of the carotid body has been proposed as a therapy for treating autonomic imbalance disorders such as hypertension and heart failure.

The carotid sinus region thus contains both baroreceptors and chemoreceptors. Excitation of the baroreceptor and the chemoreceptor nerves has opposite effects, as baroreceptor causes downregulation of sympathetic tone, while chemoreceptor excitations cause upregulation of sympathetic tone. What is needed is a way to selectively block or stimulate nerve fibers innervating the carotid sinus region, such as a way to strategically excite the baroreceptor nerves while at the same time blocking the chemoreceptor nerves.

WO 2013/018083 A2 discloses a system for treating a medical condition in a living body, comprising two subsystems, an implant subsystem and an electrical stimulation unit subsystem. The implant subsystem comprises at least one electrostimulation module, contains at least one electrically conductive electrode and, preferably, an anchoring member. The electrical stimulation unit, adapted for producing and controlling electrical waveforms, is connected to the electrodes. The implant subsystem is implanted adjacent to at least one of the following structures: the carotid sinus nerve, aortic nerve, common carotid artery, external carotid artery, internal carotid artery, carotid artery bifurcation, carotid body, aortic body or aortic arch receptors. The electrical stimulation unit is maintained outside the patient's body and is adapted to program, generate, control and deliver the electrical waveform via a wired or a wireless connection to the implant subsystem, thereby stimulating the structure it is adjacent to and treating the medical condition. Document WO-A-2013/018083 discloses the most relevant prior art.

### SUMMARY

The present subject matter may be used to stimulate desired neural pathways, and may be used to provide a depletion block on neural pathway on which it is not desired to stimulate.

An example of a system may include at least one electrode for placement on tissue in a carotid sinus region and a stimulator. The stimulator may be configured to use the at least one electrode to deliver neural stimulation to a baroreceptor region or at least one nerve innervating the baroreceptor region in the carotid sinus region to elicit a baroreflex response, and to deliver a blocking stimulation to a carotid body or at least one nerve innervating the carotid body in the carotid sinus region to inhibit a chemoreceptor response, the stimulator configured to simultaneously deliver the neural stimulation and the blocking stimulation.

An example of a method may include delivering neural stimulation to a baroreceptor region in a carotid sinus region or at least one nerve innervating a baroreceptor region to elicit baroreflex response, and delivering blocking stimulation to a carotid body in the carotid sinus region or at least one nerve innervating the carotid body to inhibit a chemoreflex response.

This Summary is an overview of some of the teachings of the present application and not intended to be an exclusive or exhaustive treatment of the present subject matter. Further details about the present subject matter are found in the detailed description and appended claims. Other aspects of the disclosure will be apparent to persons skilled in the art upon reading and understanding the following detailed description and viewing the drawings that form a part thereof, each of which are not to be taken in a limiting sense. The scope of the present disclosure is defined by the appended claims and their legal equivalents.

### BRIEF DESCRIPTION OF THE DRAWINGS

Various embodiments are illustrated by way of example in the figures of the accompanying drawings. Such embodiments are demonstrative and not intended to be exhaustive or exclusive embodiments of the present subject matter.
FIG. 1 illustrates, by way of example and not limitation, some neural tissue in a carotid sinus region.
FIG. 2 illustrates neural activity at a synapse between a nerve and another membrane.
FIG. 3 illustrates an experimental setup used to observe a presynaptic terminal depletion block.
FIG. 4 illustrates the observed relationship between the stimulus signal and the recorded ENG and EMG signals when the stimulus changes from 20 Hz to 200 Hz, and also includes the observed time to deplete the presynaptic terminal and block the synaptic junction.
FIG. 5 illustrates the relationship between the stimulus signal and the recorded ENG and EMG signals when the stimulus changes from 200 Hz to 20 Hz.
FIGS. 6A and 6B illustrate the response of a neural muscular junction to different stimulation frequencies.
FIG. 7 illustrates, by way of example and not limitation, an embodiment electrode patch placed over the carotid sinus region.
FIGS. 8A and 8B illustrate, by way of examples and not limitation, examples of a carotid sinus patch.
FIG. 9 illustrates an example of process embodiment for delivering therapy.
FIG. 10 illustrates an example of process embodiment for testing neural targets before delivering the therapy.
FIG. 11 illustrates an example of process embodiment for identifying candidate neural regions before testing and identifying a carotid region and/or baroreceptor region before delivering the therapy.
FIG. 12 illustrates an example of process embodiment, similar to FIG. 10, for testing neural targets before delivering the therapy.
FIGS. 13A-13B illustrate an example of process embodiment for testing neural targets before delivering the therapy.
FIG. 14 illustrates, by way of example and not limitation, an embodiment of a system.
FIG. 15 illustrates, by way of example and not limitation, an implantable neural stimulator and an embodiment of an external system such as a neural stimulation system analyzer.
FIG. 16 illustrates, by way of example and not limitation, a system embodiment for mapping a baroreflex region which uses an external system to communicate with an implantable device to control the process to identify the carotid body and/or baroreceptor regions.

### DETAILED DESCRIPTION

The following detailed description of the present subject matter refers to the accompanying drawings which show, by way of illustration, specific aspects and embodiments in which the present subject matter may be practiced. These embodiments are described in sufficient detail to enable those skilled in the art to practice the present subject matter. Other embodiments may be utilized and structural, logical, and electrical changes may be made without departing from the scope of the present subject matter. References to "an", "one", or "various" embodiments in this disclosure are not necessarily to the same embodiment, and such references contemplate more than one embodiment. The following detailed description is, therefore, not to be taken in a limiting sense, and the scope is defined only by the appended claims, along with the full scope of legal equivalents to which such claims are entitled.

The carotid sinus region is innervated by nerves from the autonomic nervous system (ANS), which regulates "involuntary" organs. Examples of involuntary organs include respiratory and digestive organs, and also include blood vessels and the heart. The ANS may function in an involuntary, reflexive manner to regulate glands, to regulate muscles in the skin, eye, stomach, intestines and bladder, and to regulate cardiac muscle and the muscle around blood vessels, for example. The ANS includes the sympathetic nervous system and the parasympathetic nervous system. The sympathetic nervous system is affiliated with stress and the "fight or flight response" to emergencies. Among other effects, the "fight or flight response" increases blood pressure and heart rate to increase skeletal muscle blood flow, and decreases digestion to provide the energy for "fighting or fleeting." The parasympathetic nervous system is affiliated with relaxation and the "rest and digest response" which, among other effects, decreases blood pressure and heart rate, and increases digestion to conserve energy. The ANS maintains normal internal function and works with the somatic nervous system. The heart rate and force is increased when the sympathetic nervous system is stimulated, and is decreased when the sympathetic nervous system is inhibited and the parasympathetic nervous system is stimulated. Stimulating the sympathetic and parasympathetic nervous systems can have effects other than heart rate and blood pressure. The functions associated with the sympathetic and parasympathetic nervous systems are many and can be complexly integrated with each other.

A pressoreceptive region or field is capable of sensing changes in pressure, such as changes in blood pressure. Pressoreceptor regions are referred to herein as baroreceptors. Baroreceptors are sensitive to the stretching of the wall that results from increased blood pressure from within, and function as the receptor of a central reflex mechanism that tends to reduce the pressure. Baroreflex is a reflex triggered by stimulation of a baroreceptor. Baroreflex functions as a negative feedback system, and relates to a reflex mechanism triggered by stimulation of a baroreceptors. Increased pressure stretches blood vessels, which in turn activates baroreceptor in the vessel walls. Activation of baroreceptors naturally occurs through internal pressure and stretching of the arterial wall, which excites the parasympathetic nervous system causing baroreflex inhibition of sympathetic nerve activity (SNA) and a reduction in systemic arterial pressure. An increase in baroreceptor activity induces a reduction of SNA, which reduces blood pressure by decreasing peripheral vascular resistance. Centrally mediated reflex pathways modulate cardiac rate, contractility and excitability. Stimulating a baroreflex pathway and/or baroreceptors inhibits sympathetic nerve activity, stimulates the parasympathetic nervous system and reduces systemic arterial pressure by decreasing peripheral vascular resistance and cardiac contractility. As used herein, electrically stimulating baroreceptor includes stimulating the local vasculature containing the specific nerve endings, nerve tissue including the nerve endings that innervate the baroreceptors. Stimulation of this nerve tissue near the baroreceptor causes neural signals to be sent to the central nervous system and induces a baroreflex response. The carotid sinus region also includes a distinct carotid body that includes chemoreceptors that detect changes in the levels of blood oxygen and CO₂. Excitation of the baroreceptor and the chemoreceptors nerves has opposite effects. Whereas baroreceptor stimulation causes downregulation of sympathetic tone, chemoreceptors stimulation causes upregulation of sympathetic tone. As used herein, electrically stimulating chemoreceptor includes stimulating the nerve tissue including the nerve endings that innervate the chemoreceptor. The stimulation may be neural stimulation to elicit a response or blocking stimulation to inhibit a response.

Baroreflex stimulation has been proposed for various therapies, including hypertension therapy and heart failure therapy. Hypertension is a cause of heart disease and other related cardiac co-morbidities. Hypertension occurs when blood vessels constrict. As a result, the heart works harder to maintain flow at a higher blood pressure, which can contribute to heart failure. Hypertension generally relates to high blood pressure, such as a transitory or sustained elevation of systemic arterial blood pressure to a level that is likely to induce cardiovascular damage or other adverse consequences. Hypertension has been defined as a systolic blood pressure above 140 mm Hg or a diastolic blood pressure above 90 mm Hg. Consequences of uncontrolled hypertension include, but are not limited to, retinal vascular disease and stroke, left ventricular hypertrophy and failure, myocardial infarction, dissecting aneurysm, and renovascular disease. Heart failure refers to a clinical syndrome in which cardiac function causes a below normal cardiac output that can fall below a level adequate to meet the metabolic demand of peripheral tissues. Heart failure may present itself as congestive heart failure (CHF) due to the accompanying venous and pulmonary congestion. Heart failure can be due to a variety of etiologies such as ischemic heart disease. Other therapies have been proposed as well, such as therapies to treat arrhythmias.

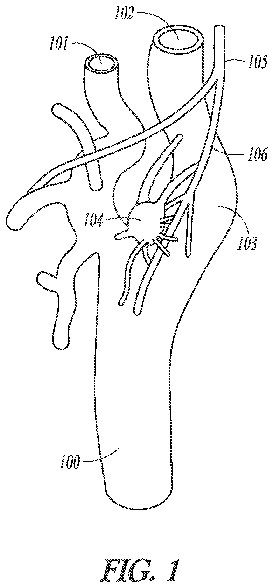

FIG. 1 illustrates, by way of example and not limitation, some neural tissue in a carotid sinus region. The illustrated physiologic structure shows the bifurcation of carotid artery, illustrating the common carotid artery 100 into an external carotid artery 101, and an internal carotid artery 102. The carotid sinus 103 is a dilated area in the bifurcation which includes many baroreceptors. Baroreceptor distribution may vary from person-to-person. However, baroreceptor appear to be more highly concentrated near the bifurcation of the interior carotid artery and external carotid artery off of the common carotid artery. Thus, some embodiments provide an orientation to stimulate the tissue area with the high concentration of baroreceptors. The carotid body 104 is located near the bifurcation and includes a cluster of chemoreceptor. FIG. 1 also illustrates nerves that innervate the region, including the glossopharyngeal nerve 105 which branches into the carotid sinus nerve 106. As provided above, baroreceptors are sensitive to changes in blood pressure. The chemoreceptors in the carotid body are sensitive to blood gas concentrations, primarily reductions in O₂ partial pressure. Both the baroreceptor and the chemoreceptors relay information to the central nervous system. Eliciting nerve traffic in neural pathways from baroreceptor can have a parasympathetic effect where their stimulation mimics the sensing of higher blood pressure causing the central nervous system to lower heart rate and blood pressure; whereas eliciting nerve traffic in neural pathways from chemoreceptor can have a sympathetic effect where their stimulation mimics a reduction in O₂ causing the central nervous system to raise heart rate and blood pressure. Baroreflex stimulation therapy may target neural tissue with a high concentration of baroreceptors while avoiding the neural tissue with chemoreceptor in the carotid body, and a chemoreflex blocking stimulation may target neural tissue with a high concentration of chemoreceptor in the carotid sinus while avoiding the neural tissue with baroreceptor.

By way of example, some embodiments may provide the chemoreflex blocking stimulation using a synaptic junction depletion block, and some embodiments may provide the chemoreflex blocking stimulation using a kHz high frequency nerve block. The kHz high frequency nerve block (e.g. 20 kHz) arrests action potentials in the captured axons, and thus prevents the action potentials from propagating through the axon. In contrast, a synaptic junction depletion block allows the action potentials to propagate through the axon but prevents the presynaptic terminal from communicating across the synaptic cleft to the postsynaptic membrane.

A brief discussion of the synaptic junction depletion block is provided below. A vagus nerve, with its complexity and innervation of many different organs, is used as example to discuss the depletion block. The depletion block may be implemented in other nerves including nerves that innervate tissue in the carotid sinus (e.g. nerves that innervate the carotid body), and including efferent and afferent nerves.

Nerve fibers, also referred to as axons, are projections from nerve cells. A nerve fiber connects a nerve cell to another nerve cell or to muscle or to gland cells at synapses. Synapses are structures that permit nerve cells to pass an electrical or chemical signal to other cells. Nerve fibers includes A fibers, B fibers, and C fibers. A fibers are the largest and, generally, the first captured as stimulation amplitude increases. A fibers can be sensory fibers (efferent) or motor fibers (efferent) that innervate muscles tissue. For example, stimulation of the vagus nerve in the cervical region may excite laryngeal muscle fibers which causing laryngeal activation which may be used as a marker for capture of the vagus nerve. B fibers are smaller and next to be captured when increasing current amplitude. These are typically efferent parasympathetic and sympathetic fibers. These B fibers may be a target for an autonomic neural stimulation therapy. C fibers are the smallest and associated with pain and other sensory information.

It has been observed that thicker nerve fibers are generally activated before thinner nerve fibers. Thick nerve fibers have longer sections of myelin sheaths between the nodes of Ranvier where the depolarization occurs and thus the change in electric field they experience is greater. By way of example, it is currently believed that the vagus nerve includes the fiber types and sizes illustrated in Table 1, and it is further believed that the majority of the fibers are C fibers.

**TABLE 1**

| Vagal Nerve Fibers | | | | |
|---|---|---|---|---|
| Fibers | Origin | Size(um) | Conduction Velocity (m/s) | Innervation |
| Aα | Motor | 13-20 | 80-120 | Larynx |
| Aγ | Motor | 5-8 | 4-24 | |
| Aα | Sensory | 13-20 | 80-120 | All organs |
| Aβ | Sensory | 6-12 | 33-75 | larynx and airways |
| Aδ | Sensor | 1-5 | 3-30 | lungs, heart |
| B (pre-g) | Efferent | 1-5 | 3-15 | stomach, pancreas |
| C (pos-g) | Efferent | 0.2-1.5 | 0.5-2 | bladder |
| C | Sensory | 0.2-1.5 | 0.5-2 | |

In general terms vagal stimulation may first capture A motor and large sensory nerves fibers, then small sensory and B parasympathetic nerve fibers. This order is a general order because fibers that are closer to the electrodes experience a stronger electric field and are activated before fibers that are further away, and further these fiber types overlap in their size. The fibers that drive heart rate down are the smallest B efferent parasympathetic fibers. These B efferent parasympathetic fibers are the smallest of the myelinated fibers, as the C fibers are unmyelinated. Neural stimulation that causes a heart rate response indicates that the B efferent parasympathetic fibers have been captured and that the other larger fiber types are also being captured.

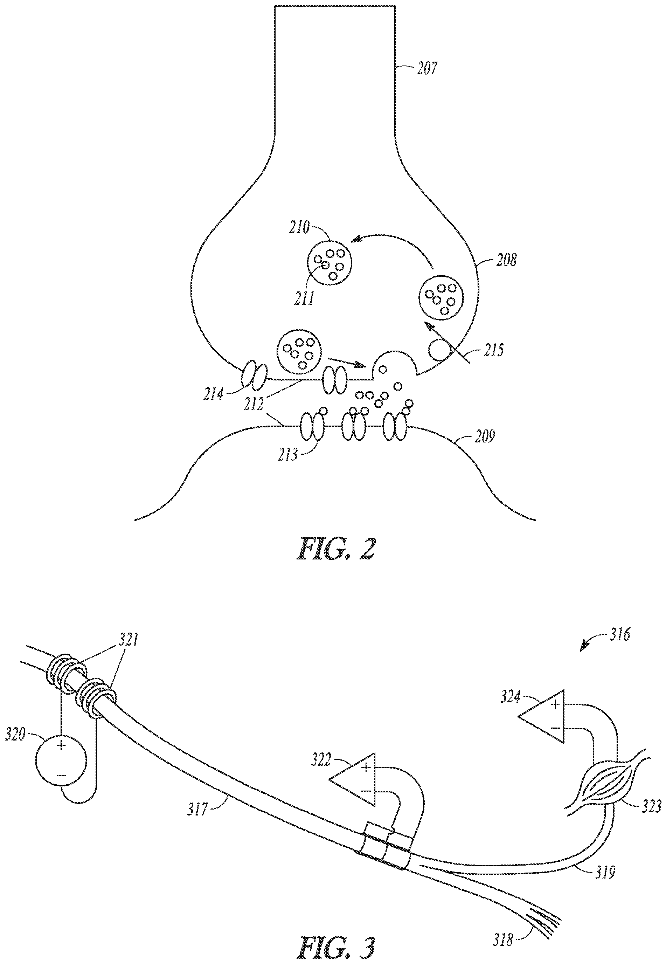

FIG. 2 illustrates neural activity at a synapse between a nerve and another membrane. An action potential propagates electrically down a nerve axon 207 until it reaches a nerve ending, which may be referred to as a presynaptic terminal 208. The presynaptic terminal communicates with a postsynaptic membrane 209 of a target cell. The target cell may be another nerve or a muscle or gland. This membrane-to-membrane junction of the presynaptic terminal and the target cell is referred to as a synapse 210. A type of synapse is an electrical synaptic junction where the presynaptic terminal electrically communicates with the postsynaptic membrane using ions or small molecules that pass through channels from one cell to the next. Another type of synapse is a chemical synaptic junction, where neurotransmitters are used to transmit between cells. The presynaptic area 208 has a large number of synaptic vesicles 210 that contain neurotransmitter chemicals 211. Action potentials that propagate to the presynaptic terminal 208 drive a chemical reaction in the presynaptic terminal that releases neurotransmitters from synaptic vesicles within the terminal into the extracellular space. This extracellular space may be referred to as a synaptic cleft 212. The neurotransmitters cross the synaptic cleft between the presynaptic and postsynaptic terminals. The neurotransmitters start a chain of reaction in receptors 213 of either the post-synaptic membrane 209 (another neuronal cell) or the muscle cells (neuromuscular junction) that trigger either the firing of an action potential in the post-synaptic neuron or the muscular contraction if the synapse ends in a neuromuscular junction. For example, where the target cell is a muscle and the synapse is a neuromuscular junction, the neurotransmitter acetylcholine (Ach) causes a rapid contraction of the target muscle cell. At a neuromuscular junction, the action potential travels to the neuromuscular synaptic junction, causing calcium ions to flow through voltage-gated calcium channels 214 which release Ach from the presynaptic terminal into the extracellular space. Postsynaptic receptors in the membrane of the target muscle cell receive the Ach. The presynaptic terminal has a neurotransmitter reuptake pump 215 that replenishes the presynaptic terminal with synaptic vesicles of neurotransmitters.

The present inventors have observed that continual communication across this synaptic cleft 212 appears to require a minimal amount of time between action potentials in the nerve, having observed that post-synaptic receptors do not trigger action potentials if the pre-synaptic action potentials arrive close to each other. Higher stimulation frequencies will generate more stimulation pulses in a given period of time, and may generate more corresponding action potentials in the nerve during the period of time. For example, a neural stimulation signal may be within a range from about 0.25 Hz to 50 Hz, or may be within a range of about 2 Hz to about 20 Hz, or may be about 20 Hz. At higher frequencies (e.g. about 100 Hz to about 1 kHz), it was observed that the presynaptic terminal was unable to communicate across the synaptic cleft even though action potentials continued to propagate through the axon. This inability of the presynaptic terminal to communicate may be referred to as a depletion block. The frequencies used to obtain this depletion block are lower than the high frequency (greater than 1 kHz) AC nerve block that would block action potentials from propagating down the nerve. At frequencies higher than 1 kHz, for example, the stimulation blocks the nerve from conducting the action potentials. In contrast, the depletion block is delivered at frequencies below 1 kHz and thus does not stop the action potentials from propagating down the nerve to the presynaptic terminal, but rather depletes the presynaptic terminal so it is no longer able to communicate across the synaptic cleft to receptors of another cell.

FIG. 3 illustrates an experimental setup 316 used to observe a presynaptic terminal depletion block. Although the experiment was set up to observe a depletion block in a cervical vagus nerve, the underlying principles that cause the block are applicable to other nerves including efferent nerves as well as afferent nerves. Thus, a depletion block may be applied to the nerves that innervate the carotid body. A cervical vagus nerve 317 branches into the thoracic branch 318 and the recurrent laryngeal nerve 319. The illustrated experimental setup was used to stimulate the cervical vagus nerve 317 using a current source 320 and helical electrodes 321 in a bipolar arrangement, to monitor neural activity before the cervical vagus nerve 317 branches into the recurrent laryngeal nerve branch 319 and the thoracic branch 318 using an electroneurography (ENG) monitor 322, and to monitor vibration of the laryngeal muscles 323 using an electromyography (EMG) monitor 324. This set up was used to observe that action potentials from depletion block stimulation were still sensed by the ENG, but laryngeal vibrations were not sensed by the EMG 218. Thus, it could be concluded that the depletion block stimulation blocked the ability of the presynaptic terminal to communicate across the synaptic cleft.

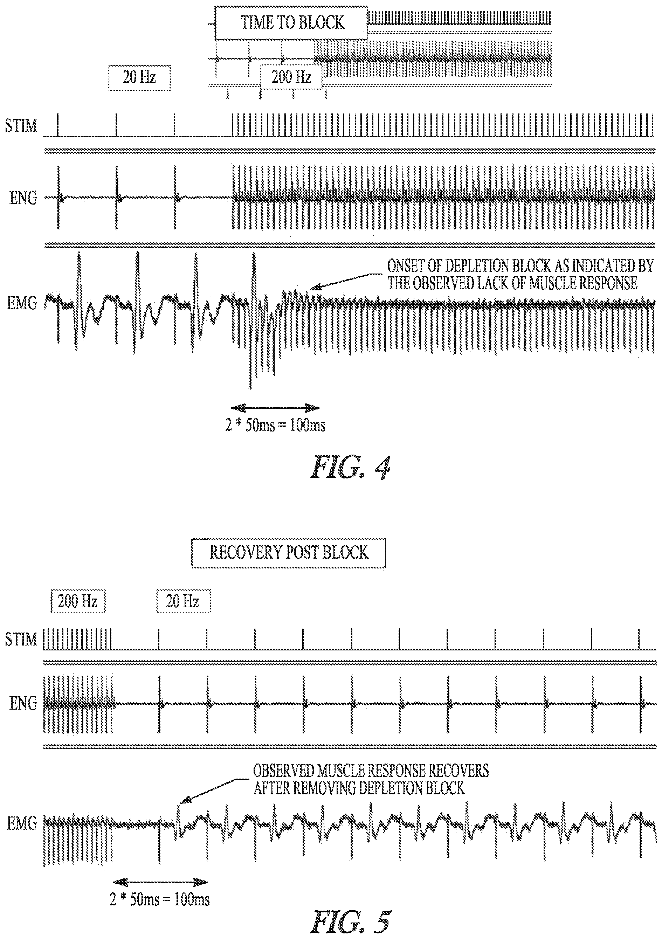

FIG. 4 illustrates the observed relationship between the stimulus signal and the recorded ENG and EMG signals when the stimulus changes from 20 Hz to 200 Hz, and also includes the observed time to deplete the presynaptic terminal and block the synaptic junction. During the 20 Hz stimulation, both the ENG and EMG signals follow the stimulus signal. The high peaks in both ENG and EMG signals reflect the stimulation artifact. However, during the 200 Hz stimulation, the ENG response is still present after the stimulus signal but the EMG signal quickly subsides after an onset response of about 100 ms. After a brief transitional period after the stimulus changes to 200 Hz, only the artifact from charge-balancing is seen in the EMG waveform. Thus, the axons in the nerve continue to be active by propagating action potentials, but the communication across the synaptic cleft is reduced or stopped after the presynaptic terminal has been depleted from its ability to communicate across the synaptic cleft. As illustrated, this synaptic junction block occurs very quickly (e.g. 50 to 100 ms after the 200 Hz signal is applied), as soon as the propagated pulses received at the presynaptic terminal deplete the presynaptic terminal from its ability to communicate. It does not appear that the physiological reuptake process that restores neurotransmitters and/or calcium in the presynaptic terminal can keep up with the transmission of the neurotransmitters from the 200 Hz stimulation. An onset period can also be observed after the frequency shift where the muscles appears to contract strongly for a short time and then relaxes. Table 2 shows the time needed for the muscles to stop contracting.

FIG. 5 illustrates the relationship between the stimulus signal and the recorded ENG and EMG signals when the stimulus changes from 200 Hz to 20 Hz. The synaptic junction block occurs when the stimulus is delivered at 200 Hz. During this time, the ENG is still present following the stimulus artifact signal but the EMG response is not present. This indicates that the stimulus is capturing the nerve and causing action potentials to propagate through the axon. Every pulse in the stimulation causes a respective action potential in the nerve fiber. However, the laryngeal muscle is not stimulated because of the presynaptic terminal depletion that causes the synaptic junction block. The 200 action potentials per second deplete the ability of the presynaptic terminal to communicate across the synaptic cleft. When the stimulus changes from 200 Hz to 20 Hz, however, the ENG response continues to be present following the stimulus pulse as every pulse in the stimulation causes a respective action potential in the nerve fiber. The EMG reappears right after the stimulus pulse just after a brief transitional period after the stimulation frequency changes to 20 Hz. The ability of the presynaptic terminal to communicate across the synaptic cleft is not depleted by 20 pulses per second. Thus, as illustrated, the synaptic junction block can be removed very quickly (e.g. 50 ms to 100 ms after the signal changes from 200 Hz to 20 Hz signal), which is believed to reflect the physiological response time for restoring neurotransmitters and/or calcium in the presynaptic terminal.

Table 2 illustrates that certain frequencies can turn the depletion block of the synaptic junction on/off more quickly than other frequencies. Data suggest that frequencies greater than about 200 Hz provide a fast depletion block, whereas frequencies between about 100 to about 150 Hz provides slower depletion blocks. Frequencies below 100 Hz tend not be effective to provide the depletion block, as those frequencies do not exceed the ability of the presynaptic terminal to restore its ability to communicate from the presynaptic terminal across the synaptic cleft to the target cell. In a neural muscular junction, for example, frequencies less than about 100 Hz cause tetanic contraction; frequencies between about 100 to about 150 Hz causes a 90% depletion block in about 10 seconds to 4 seconds; a frequency between about 200 Hz to 1000 Hz causes a 90% depletion block. Nerve conduction block where the stimulation arrests the action potentials propagating down the nerve has been observed at frequencies as low as 1kHz but more typically between 5-10 kHz.

**TABLE 2**

| | | Time to 90% Block (sec) | | Percentage of unblocked EMG (%) | |
|---|---|---|---|---|---|
| | Freq (Hz | mean | stdev | mean | stdev |
| Activation | 40¹ | - | - | 110 | 13.18 |
| | 70¹ | - | - | 39 | 8.42 |
| Slow Block | 100^{*,2} | 10.74 | 2.2 | 8.2 | 3.77 |
| | 130¹ | 9.33 | 0.55 | 4.38 | 1.06 |
| | 150² | 4.43 | 2.59 | 3.88 | 1.13 |
| Fast Block | 200² | 0.53 | 0.16 | 2.25 | 1.04 |
| | 260¹ | 0.16 | 0.05 | 0.75 | 0.89 |
| | 300² | 0.13 | 0.05 | 1.13 | 1.13 |
| | 400¹ | 0.14 | 0.05 | 0.63 | 0.74 |
| | | | | | |

| | | | | | |
|---|---|---|---|---|---|
| Randomized study; n=8 (100hz: n=5), data from 2 * N=1 | | | | | |

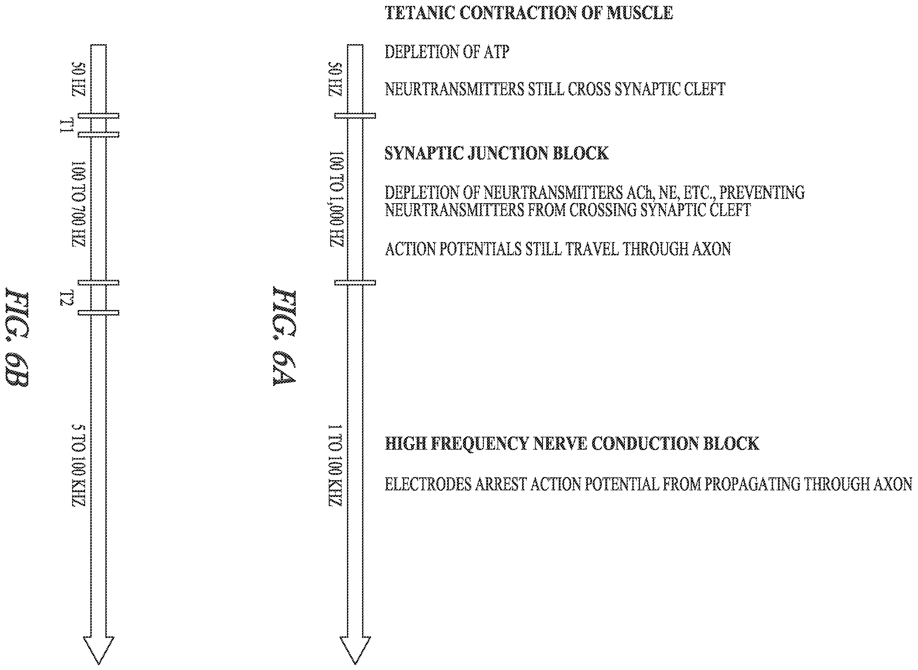

FIG. 6A illustrates the response of a neural muscular junction to different stimulation frequencies. The neutral muscular junction is a type of synaptic junction where an axon in a nerve communicates with muscle. Stimulation of axons within a range generally below 100 Hz (e.g. about 50 Hz) may cause a tetanic contraction of the muscle. Eventually, the muscle may fatigue and no longer respond to additional stimulation. The presynaptic terminal is depleted from its ability to communicate across the synaptic cleft at stimulation frequencies within a range from about 100 Hz to about 1kHz. This frequency of the stimulation signal is outside of the ability of the physiological system to trigger the muscular contraction, as the frequency may cause the action potentials to arrive faster than the neurotransmitters and/or calcium can be replenished for subsequent action potentials in the stimulation. The observed block is attributable to a depletion of the junction but not fatigue of the muscle. Thugs, a benefit of the depletion block applied to neural muscular junctions is that the depletion block does not cause muscle fatigue or tetanic contraction. The neuromuscular depletion block is quickly reversible by stopping stimulation.

It is noted that FIG. 6A is a simple illustration of frequency ranges, and that these ranges may vary for different applications. FIG. 6B provides another illustration of a response of a neural muscular junction to different stimulation frequencies. FIG. 6B illustrates a transition period T1 between the activation and depletion block ranges. Transition period T1 may depend on the transmitter and the synaptic end-organ, and may range from about 70 to 130 Hz. FIG. 6B also illustrates a transition period T2 between the depletion block and conduction block ranges that may provide a combined depletion and conduction block.

Some characterizations of depletion block, combined depletion and conduction block, and high frequency kHz conduction blocks are provide below. For example, a depletion block has a lower frequency and thus lower power requirements, has a relatively fast block (< 100 ms) and a relatively fast recovery (<100 ms over 50% and 10 seconds 100%). For example, a combined depletion and conduction block (e.g. around 1 kHz may block slow fibers extremely fast due to conduction block, may be initiated with a high kHz frequency and then lowered to keep the block at lower frequencies, may block slower fibers in less than 7 ms, and may have a faster recovery than the higher frequency kHz blocks. For example, a high frequency kHz conduction block is fast (e.g. on: < 7ms an off: <10 ms), but is more energy intensive due to higher frequencies and current requirements.

For example, a kHz conduction block may be observed with a lower boundary of about 1 kHz to 5 kHz rather than the simply illustrated 1 kHz. Additionally, the upper boundary of a depletion block may be about 2 kHz rather than the simply illustrated 1 kHz. Further, the frequencies for which stimulation transitions from depletion to conduction depends on the nerve fibers and end plate. Fast α-fibers have higher conduction and firing rates, so they will not necessarily block at 1kHz, and slower fibers will block at lower frequencies (e.g. 600 Hz). Thus, there may be a nerve stimulation frequency band within which most fibers can be activated, a depletion block frequency band for which most fibers may be depleted, and a kHz conduction block frequency band for which most fibers have their action potentials blocked. By way of example, the nerve stimulation frequency band may extend up to about 50 Hz, the depletion block frequency band may extend between about 100 Hz to about 700 Hz, and the kHz conduction block frequency band may extend from about 5 kHz to 100 kHz. There may be transition frequencies between the bands, such as a transition between about 50 Hz to about 100 Hz or between about 70 Hz to 130 Hz for example and another transition between about 700 Hz to about 5 kHz. The response of the nerve to the stimulation frequency appears to depend on the transmitter and the synaptic end organ. Thus, different types of fibers may react differently for frequencies within the transition frequencies. By way of example, one frequency may cause an activation or neural stimulation of some fibers, and cause a depletion block in other fibers. The stimulation may be limited to specific fibers by the diameter or origin of the fibers or the location of the electrodes. For example, a frequency of the depletion block stimulation may be found to discriminate between afferent and efferent nerve fibers, or to discriminate between different fibers that emit different types of neurotransmitters. Such a frequency capable of providing both depletion block and activation/stimulation may be found in a transition region, but also may be found in one of the frequency bands such as within the depletion block frequency band.

Although the response to different frequencies may and is expected to change from application to application, the stimulation parameters for delivering a depletion block are expected to be available in current devices at reasonable energy consumption costs. In the study illustrated in the table where stimulation is provided at a pulse width of 300 µs, A-fibers were blocked at 2 mA, 200 Hz while still exciting B fibers that drove heart rate down at 5mA, 20 Hz.

As illustrated in Table 2, the speed of the depletion block depends on the frequency of the stimulation, where higher frequencies within the range of about 100 Hz to about 1 kHz provide the neurotransmitter block more quickly than the lower frequencies within that range. According to some embodiments, the depletion block may be implemented by a process that initiates the depletion block at a relatively high frequency (e.g. about 200 Hz to 400 Hz) to achieve fast depletion (e.g. about 50 ms or less), and then subsequently lower the frequency of the depletion block stimulation to about 100 Hz to maintain the block. As the lower frequency stimulation delivers fewer pulses, the lower frequency depletion block is more energy efficient than the higher frequency depletion block. If the depletion block was started at about 100 Hz rather than 200 Hz, it would take longer to achieve the depletion block. Based on current observations, it is believed that the depletion block at 100 Hz will take about 5 seconds to 10 seconds. The use of two (or more) stages of frequencies can be used to obtain benefits of each frequency, such as inducing depletion block relatively quickly using a one frequency and then maintaining depletion block relatively efficiently using another frequency.

Different neurotransmitters may have different frequency thresholds for depletion. By way of illustration, a threshold frequency for depleting a first neurotransmitter may be at a first frequency within the range of 100 Hz to 1 kHz, and a threshold frequency for depleting a second neurotransmitter may be at a second frequency, higher than the first, within the range of 100 Hz to kHz. Thus, various embodiments may use frequency to discriminate between different types of axons. For example, if a frequency of the stimulation is set between the first and second frequency thresholds, then the stimulation will cause a depletion block for a first type of axon by depleting the first neurotransmitter, but will not cause a depletion block for a second type of axon as the second neurotransmitter is not depleted by the stimulation. This may be used to discriminate between different types of neural targets in the carotid body. Various embodiments may use a depletion block at the synaptic junction to provide selective fiber communication. A depletion block may be limited to specific fibers by diameter or origin or location to the electrode. The amplitude of the depletion block pulses can be controlled to be greater than only the stimulation threshold for only some of the nerve fibers. Thus, although all fibers may be with other pulses that causes action potentials to propagate, the presynaptic terminal for some of the fibers are quickly depleted from their ability to communicate across the synaptic junction because the frequency of the stimulation causes the depletion block. Various stimulation waveforms may be used including non-sinusoidal or sinusoidal waveforms. Non-sinusoidal waveforms may include rectilinear pulses, charge balanced waveforms that may include biphasic rectangular pulses, quasi-trapezoidal for unidirectional applications, and pulsed triangular.

Neural stimulation that elicits nerve traffic and a desired physiological response as part of neural stimulation therapy may be referred to as a low frequency stimulation (e.g. about 20 Hz or within a range of about 5 Hz to about 50 Hz); whereas in comparison a depletion frequency may be referred to as high frequency (e.g. about 200 Hz or within a range of about 100 Hz to about 1 kHz). A range of about 100 Hz to about 1 kHz includes a range from 100 Hz to 1 kHz, as well as frequencies effectively near that range to provide the depletion block. Even higher frequency in the kHz range, such as maybe used to block propagation of action potentials, may be referred to as kHz high frequency. The stimulation at the lower frequencies that is effective in activating nerve fiber(s) to deliver a nerve stimulation therapy may be referred to herein simply as "nerve stimulation" or "neural stimulation;" whereas the stimulation at the higher "depletion" frequencies may be referred to herein simply as a "depletion block stimulation." A "high amplitude, low frequency" (HALF) stimulation signal may exceed a stimulation threshold and thus may be used to recruit both small and big fibers. As such, a HALF signal may be used to obtain the desired effect of the stimulation by capturing all the necessary A sensory and B efferent fibers. A "small amplitude, high frequency" (SAHF) stimulation signal may be set at an amplitude that it only exceeds a stimulation threshold and thus only recruits the bigger fibers with the lower stimulation threshold while leaving the smaller fibers still excitable with the HALF stimulation. The depletion block stimulation cancels the effectiveness of all signals that are evoked at lower frequencies (e.g. 20 Hz) with the same or lower amplitude. SAHF may be used to achieve the neurotransmitter depletion block of the large fibers which are the fibers with relatively low stimulation thresholds but not the smaller fibers which are the fibers with relatively high stimulation thresholds. In some embodiments, the higher frequency depletion block stimulation may be delivered using the same or approximately the same high amplitude as the low frequency stimulation to reduce or modulate the effect of the applied therapy using the low frequency stimulation.

The current amplitude and the pulse width control whether an axon is depolarized, and the frequency of the stimulation controls whether the neurotransmitters are depleted at the nerve ending. The current amplitude and pulse width may be controlled to select only larger fibers for the depletion block. For example, the current amplitude and pulse width may be controlled to deplete the A fibers and not the smaller fibers, or may be controlled with higher amplitudes and/or wider pulse widths to deplete both A and B fibers.

By way of example and not limitation, a full neurotransmitter block for intended fibers may be ensured by acquiring a recruitment curve. The recruitment curve may identify the activation threshold and saturation threshold for the neural target. The recruitment curve may be specific to an individual patient, may illustrate an increase in activity with increasing current amplitude, and may then illustrate a plateau where the activity does not significantly increase with increasing current amplitude. The activation threshold reflects where the nerve activity begins to increase with increasing current amplitude, and the saturation threshold reflects where the nerve activity does not significantly increase in response to further increases in current amplitude. The current amplitude for the depletion block stimulation may be determined based on the activation threshold, as it may be set at a margin higher than the activation threshold. The saturation threshold indicates a threshold where all or almost all of the nerve fibers propagate action potentials. The current amplitude for the depletion block stimulation may be determined based on the saturation threshold of the fibers that are intended to be blocked. By way of example, the amplitude of the depletion stimulation signal may be set at approximately the saturation threshold of the fibers that are intended to be blocked, or may be set at a margin higher than the saturation threshold of the fibers, or may be set at a margin lower than the saturation threshold to provide a partial block.

A procedure can be implemented to determine each individual patient's selective fiber stimulation therapy profile, as there may be patient variation or variations resulting from electrode spacing from nerves fibers. The particular procedure will depend on the particular neural target that is stimulated. For example, if a baroreceptor region or a nerve that innervate the baroreceptor region is targeted, the patient's selective fiber stimulation therapy profile may be determined by observing blood pressure and heart rate fluctuation. If a carotid body or a nerve that innervate the carotid body, the patient's selective fiber stimulation therapy profile may be determined by observing fluctuations in respiration. Thus, various embodiments for providing a depletion block may first find an activation threshold and saturation threshold for a neural target. The current amplitude may be selected to be above the saturation threshold of the neural target, and the frequency may be selected for a given application to be high enough (e.g. 200 Hz) to quickly deplete the presynaptic terminal of its ability to communicate across the synaptic cleft to provide an effective depletion block for that application. The procedure may transition the frequency of the stimulation while monitoring the physiological effects to transition between different types of block (e.g. transition between depletion block and kHz conduction block), or to improve efficiency, or to improve time constants (e.g. onset / restoration), or to find a desired frequency and location that both activates some nerve fibers and also provides a depletion block for other nerve fibers.

Some embodiments may ramp up stimulation. Ramping up the stimulation may provide a graded block that may make the stimulation more tolerable. In a neural muscular junction depletion block, for example, the ramped stimulation may reduce the force of the one initial muscle activity at start of stimulation by creating an initial period of graded block. Some embodiments may change the frequency of stimulation signal during the block. Thus, higher frequency stimulations may be used to quickly obtain the block, and then lower frequency stimulation may be used to maintain the block that was previously obtained. For example, an initial frequency (e.g. 260 Hz) may be used to quickly achieve depletion block followed by a second frequency (e.g. 130 Hz) to maintain the depletion block. The frequency of stimulation is related to how long for complete or 90% depletion block. For example, frequencies within the range of about 100 to about 150 Hz provide a 90% depletion block in about 10 to 4 seconds, and frequencies within the range of about 200 to 1000 Hz provides a 90% depletion block less than one second (e.g. on the order of milliseconds). Frequencies greater than 1 kHz start to enter into nerve conduction block.

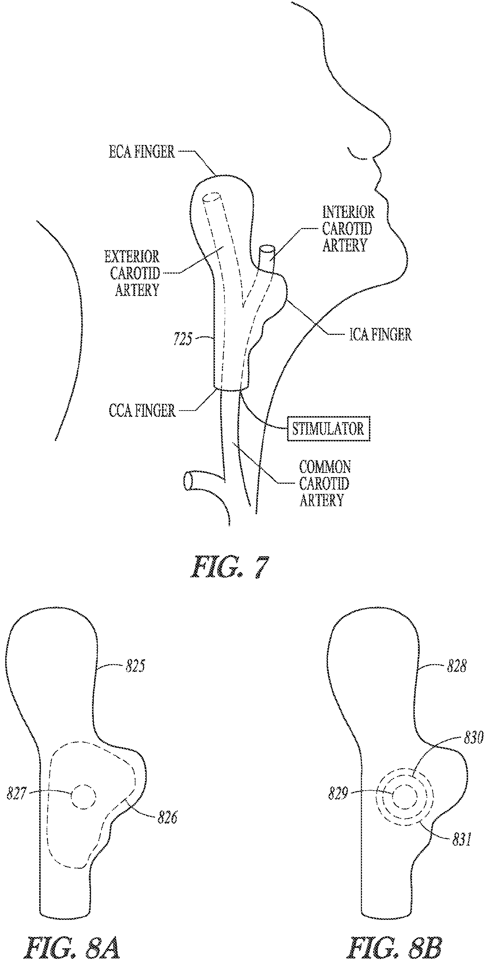

FIG. 7 illustrates, by way of example and not limitation, an embodiment electrode patch placed over the carotid sinus region. The patch 725 may function as a substrate for the electrodes. The electrodes may be attached to the patch or formed with the patch. The patch 725 may be attached to a stimulator. The shape of the patch 725 may be designed to reduce the invasiveness of the surgical procedure, while still covering neural targets in the carotid sinus region. The patch 725 may have protrusions, which may be referred to as fingers. The patch 725 may be configured to lie over the carotid sinus, with the fingers designed to extend along the common carotid artery, the interior carotid artery and the exterior carotid artery. A common carotid artery (CCA) finger runs along the common carotid artery, an interior carotid artery (ICA) finger runs along the interior carotid artery, and the exterior carotid artery (ECA) finger runs along the exterior carotid artery. The length of the patch from the CCA edge to the ECA edge may be within a range of about 2 cm to about 4 cm, and the length of the ICA finger of the patch may be within a range of about 0.5 cm to about 2 cm. The width of the ECA finger may be about 0.5 cm to about 1 cm, the width of the ICA finger may be about 0.5 cm to about 1.5 cm. In some embodiments, the distance between electrodes may be within a range of about 0. 5 mm to 2 mm edge to edge.

Other substrates may be used to position a pattern of electrodes over a carotid sinus region. For example, the patch may include electrodes over a silicon sheath. The pattern of electrodes may be a simple array of electrodes with a rows and columns of electrodes.

The system may be designed to electrically connect to select electrode(s). The system may include switches under the control of a controller, and/or may include a mux on or near the carrier (e.g. patch) of the electrodes. By way of example, the electrode configuration may be a matrix (e.g. 5 by 5 electrode contacts) with some of the contacts used for stimulation while other contacts are used for blocking. The blocking electrodes / signal may be controlled to control whether action potentials that result from stimulation pass to various end organs. Some embodiments may employ a mux to control where each type of stim goes. Some embodiments may block and stimulate with the 200 Hz (depletion) as well as the∼1kHz (combined) as well as the full KHFAC nerve block. This block at this stimulation may be limited to specific fibers by diameter or origin or location to the electrode.

Some examples may implant an electrode patch, and some examples may use an external electrode patch that may be placed on the skin over the carotid sinus region. An external stimulation system and external patch may be used to screen potential patients to determine suitability for an implanted device. Implanted patches with multiple electrodes may allow clinician and/or patient to adjust the locations of and the field for the stimulation and block. For example, activation thresholds and selectivity may change if the patient changes position (e.g. lies down on the patch).

FIG. 8A illustrates, by way of example and not limitation, an example of a carotid sinus patch 825 with electrode pattern region 826. The electrode pattern region 826 can include a number of electrodes configured in an array or other pattern for placement over neural targets in the carotid sinus region. Modeling information suggests that it is the tissue directly under the cathode that gets the greatest amount of energy. Thus, it may be desirable to locate the cathode of the stimulation near a neural tissue hotspot. U.S. Provisional Patent Application 61/836,431 filed on June 18, 2013 and entitled System and Method for Mapping Baroreceptors relates to mapping a baroreceptor region and position a cathode on a baroreceptor hotspot. U.S. Provisional Patent Application 61/836,431 is incorporated by reference herein in its entirety. The location of the anode appears to be less significant, and may be positioned away from potential cathodes for the stimulation configuration. Therefore, electrodes in the mapping region may be configured to be potential cathodes, and the anode(s) may be on the ECA finger.

As the surgeon is capable of visually identifying the carotid body during surgery, some embodiments may have blocking electrode(s) in the patch, such as at region 827. The surgeon may place this region 827 containing blocking electrode(s) on or near the carotid body during the placement of the patch in the carotid sinus region. The blocking electrode(s) may be used to deliver the blocking stimulation to inhibit the chemoreflex, Electrode(s) on the patch around the region 827 then may be used to deliver baroreflex stimulation. Some embodiments may search for a carotid body region and/or search for baroreceptor region. A test stimulation (e.g. a kHz blocking stimulation, a depletion block stimulator or neural stimulation) may be applied to a test region within a carotid sinus region, and a physiological parameter(s may be monitored to determine if the test region is a carotid body or a nerve that innervate the carotid body, or is a baroreceptor region or a nerve that innervate the baroreceptor region. For example, the blocking stimulation may be applied using different electrode combinations until a desired physiological response is observed, and baroreflex stimulation may be applied using different electrode combinations until a desired physiologic response is observed. Some embodiments may sense transthoracic impedance to sense breathing parameters indicating chemoreceptor effects to the blocking stimulation. Some embodiments may sense heart rate indicating baroreflex effects to the neural stimulation. For example, an accelerometer may be attached to the stimulation lead implanted in the carotid sinus region. This accelerometer may be used, for example, to sense blood pulses or heart sounds propagating up the carotid artery. Furthermore, the accelerometer may be used to sense breathing sounds and/or airflow and may be an indication of breathing rate. This search may start with electrode(s) near the region 827 placed on or near the carotid body. In addition to using some electrodes on the patch to provide a blocking stimulation to the carotid body and using other electrodes on the patch to provide a neural stimulation, some embodiments may use still other of electrodes on the patch as a physiologic sensor.

FIG. 8B illustrates, by way of example and not limitation, an example of a carotid sinus patch 828 with an electrode region 829. The electrode region 829 may include at least one electrode for placement on tissue in a carotid sinus region. A stimulator may use the at least one electrode to deliver neural stimulation to a baroreceptor region or a nerve innervating the baroreceptor region in the carotid sinus region to elicit a baroreflex response, and to deliver a blocking stimulation to a carotid body or a nerve innervating the carotid body in the carotid sinus region to inhibit a chemoreceptor response, the stimulator configured to simultaneously deliver the neural stimulation and the blocking stimulation. A stimulator may use a stimulation vector to deliver the blocking stimulation and to use the same stimulation vector to deliver the neural stimulation. For example, the electrode region 829 may include a single electrode used to deliver unipolar stimulation. An electrode on the housing of the stimulator may complete the current path. A stimulation vector is provided by the single electrode and the electrode on the housing. The amplitude of the blocking stimulation may be lower than the amplitude of the neural stimulation. Thus, if electrode region 829 is placed over or near the carotid body, the blocking stimulation may be used to capture the neural tissue in the carotid body region. The neural stimulation may be delivered using the stimulation vector with a first current amplitude to capture neural tissue in region 830, or may be delivered with a second higher current amplitude to capture neural tissue in region 831. The neural tissue in regions 831 and 832 may include tissue that includes a baroreceptor region or a nerve that innervate the baroreceptor region. For example SAHF may be used to provide the depletion block under region 829, and HALF may be used to provide the neural stimulation under regions 830 or 831. The stimulator may use two electrodes on the patch to deliver bipolar stimulation providing a stimulation vector that is effective in capturing neural tissue under region 829. The stimulation vector provided by the bipolar stimulation may be used to capture neural tissue under region 829 and deliver a depletion block, and the same stimulation vector provided by the bipolar stimulation may be used to capture neural tissue under regions 830 or 831.

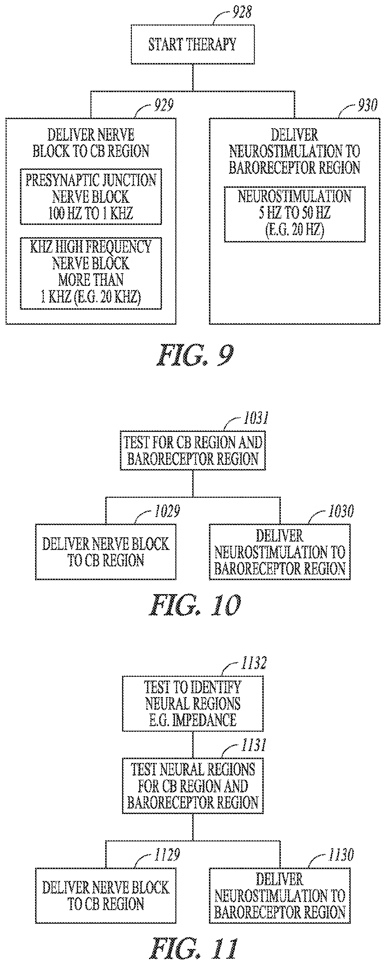

FIG. 9 illustrates an example of process embodiment for delivering therapy. The therapy may begin at 928, and a nerve block may be delivered to the carotid body region at 929 to inhibit a chemoreflex. This nerve block may be a presynaptic junction nerve block, with stimulation frequencies between 100 Hz and 1 kHz as discussed above, or may be a Hz high frequency nerve block, with stimulation frequencies over 1 kHz such as 20 kHz). One benefit of the depletion block is that it has a much lower frequency and therefore is more energy efficient than the kHz high frequency nerve block. Some embodiments may also deliver a neurostimulation to baroreceptor region(s) to stimulate a baroreflex 930. This stimulation may be within a range of 0.25 Hz to 50 Hz, such as at 20 Hz for example. The blocking stimulation and neurostimulation may be delivered together simultaneously, or may be delivered intermittently and at different times from each other. The blocking stimulation and neurostimulation may be programmed to function independently without coordination with the other, or may be programmed to be performed in a coordinated matter with each other.

Some embodiments may perform a test to identify the carotid body region before delivering the blocking stimulation to inhibit the chemoreflex, and some embodiments may perform a test to identify the baroreceptor region before delivering the neurostimulation to elicit a baroreflex. FIG. 10 illustrates an example of process embodiment for testing neural targets before delivering the therapy. Thus, for example, some embodiments deliver stimulation to the carotid body at 1031 and observe for a desired physiological response. If the applied test stimulation elicits a chemoreflex, the expected physiological response is expected to be consistent with an increase in sympathetic activity. If the applied test stimulation blocks a chemoreflex, the expected physiological response is expected to be consistent with a decrease in sympathetic activity such as may be deflected by breathing patterns. If the expected response is not observed, the electrode(s) may be moved or different electrode(s) may be selected for the test. Some embodiments may deliver stimulation to a baroreceptor region at 1031 and observe for a desired physiological response. If the applied test stimulation elicits a baroreflex, the expected physiological response is expected to be consistent with an increase in parasympathetic activity. If the applied test stimulation blocks a baroreflex, the expected physiological response is expected to be consistent with a decrease in parasympathetic activity. If the expected response is not observed, the electrode(s) may be moved or different electrode(s) may be selected for the test. After the tests are performed to identify the regions, the nerve block may be delivered to the CB region 1029 and/or the neurostimulation may be delivered to the baroreceptor region 1030.

Some embodiments may test for neural regions to identify candidate neural regions for stimulation, and then test these identified candidate neural regions to distinguish between carotid body and baroreceptor regions. For example, some embodiments may test the electrical characteristic of tissue (e.g. impedance) to distinguish neural tissue from other tissue in the region. U.S. Provisional Application 61/918269, filed December 19, 2013 and entitled "System and Method for Locating Neural Tissue" describes locating neural tissue, and is hereby incorporated by reference in its entirety. FIG. 11 illustrates an example of process embodiment for identifying candidate neural regions 1132 before testing and identifying a carotid region and/or baroreceptor region 1131 before delivering the therapy 1129 and/or 1130.

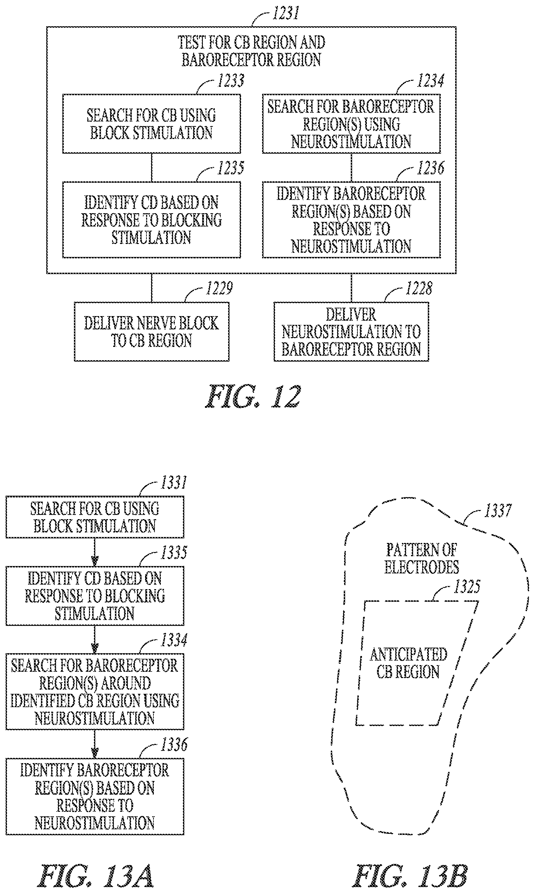

FIG. 12 illustrates an example of process embodiment, similar to FIG. 10, for testing neural targets before delivering the therapy. In FIG. 12, the test for a carotid body region and a baroreceptor region(s) 1231 includes an algorithm for searching for the carotid body using blocking stimulation 1233 and for searching for the baroreceptor region(s) using neurostimulation 1234. For example, the algorithm can step through potential electrode combinations on the patch to determine which electrode combinations are effective in inhibiting a chemoreflex when the blocking stimulation is applied (corresponding to the carotid body) and which electrode combinations are effective in eliciting a baroreflex when the neurostimulation is applied (corresponding to baroreceptor region(s)). At 1235, a physiological response to the blocking stimulation can be monitored to identify whether the carotid body has been found using a particular electrode combination. At 1236, a physiological response to the neurostimulation can be monitored to identify whether a baroreceptor region has been found using a particular electrode combination. The search for the carotid body may be performed at different times or may be performed simultaneously with the search for the baroreceptor region. The strength of the blocking stimulation field and strength of the neurostimulation field may be controlled by controlling the applied stimulation current, for example, such that only the neural tissue closest to the active electrode(s) are captured. The nerve block may be delivered to the identified carotid body 1229 and/or the neurostimulation may be delivered to the identified baroreceptor region 1228.

FIGS. 13A-13B illustrate an example of process embodiment for testing neural targets before delivering the therapy. This example performs a smarter search, as it assumes that the physician can place a target area of the patch over the carotid body as the physician can visually identify the carotid body. The method first searches for the carotid body region 1331 within the anticipated carotid body region 1337 on the patch 1325 and then identifies the carotid body based on a response to the stimulation 1335 (e.g. blocking stimulation reduces chemoreflex or neural stimulation increases chemoreflex). The search for the carotid body may be expanded if it is not found within the anticipated carotid body region of the patch. Once this location is determined, the algorithm can than proceed with searching for baroreceptor regions 1334 around the identified carotid region to identify the baroreceptor region(s) based on a response to the stimulation 1336 (e.g. neurostimulation elicits a baroreflex or blocking stimulation decreases baroreflex).

A method may include simultaneously applying electrical blocking and excitation current to the carotid body and/or to baroreceptor regions or carotid sinus nerve. A physiologic detector may be used to sense parameters of breathing patterns. The electrical blocking stimulation may be modulated until the breathing patterns are slowed. A physiologic detector may be used to sense parameter indicative of blood pressure. The electrical neural stimulation may be modulated until blood pressure is lowered. This method may be repeated at programmed times.

Different neurotransmitters may have different frequency thresholds for depletion. This may be used to discriminate between different types of neural targets in the carotid body. The depletion stimulation may be set to be between a frequency threshold for depleting neurotransmitters in the axons innervating the baroreceptor region and another frequency threshold for depleting neurotransmitters in the axons innervating the carotid sinus region to distinguish between the regions. The stimulation may cause a depletion block (or a faster depletion block) for the axons that have the lower frequency threshold for their neurotransmitters, but not the axons that have the higher frequency threshold.

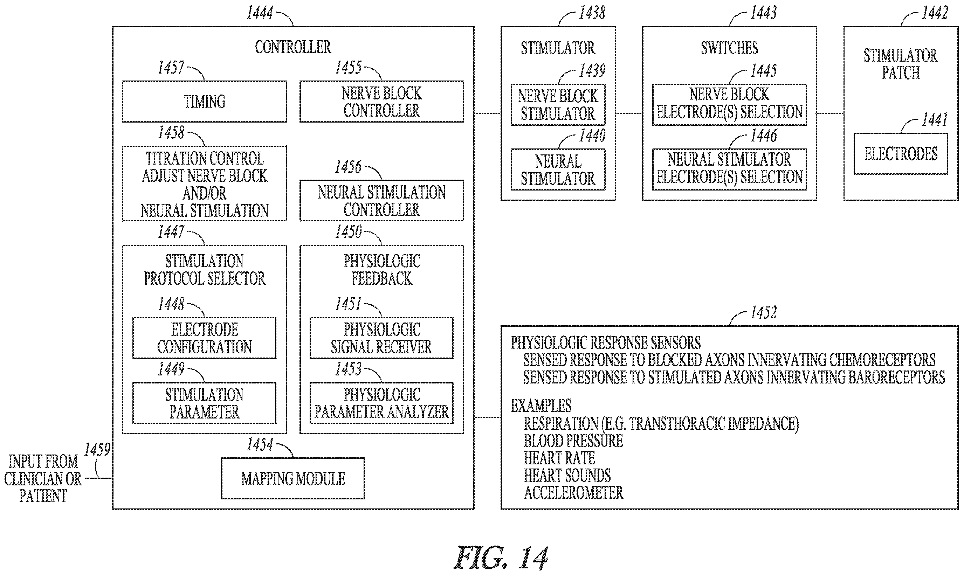

FIG. 14 illustrates, by way of example and not limitation, an embodiment of a system. In some embodiments, the system may include a stimulator 1438 which may include a nerve block stimulator 1439 configured to deliver a nerve block and a neural stimulator 1440 configured to deliver neural stimulation. The system may be configured to deliver stimulation through electrodes 1441 to neural targets in the carotid sinus region. For example, the system may be configured to deliver a nerve block to the carotid body to inhibit a chemoreflex and may further be configured to deliver neural stimulation to baroreceptor region(s) to elicit a baroreflex response.

Some embodiments may provide a pattern of electrodes on a stimulation patch 1442. Switches 1443 may be controlled by a controller 1444 to connect the nerve block stimulator 1439 to a selection of electrodes 1445 for use in the nerve block, and may further be controlled by the controller 1444 to connect the neural stimulator 1440 to a selection of electrodes 1446 for use in the neural stimulation.

The illustrated controller 1444 may include a stimulation protocol selector 1447. The stimulation protocol selector 1447 may include an electrode configuration selector 1448, which may work with the module of switches 1443 to control switching. The stimulation protocol selector 1447 may also include a stimulation parameter selector 1449 which may work with the stimulator 1438 to control the stimulation parameters. Such parameters may include the amplitude, pulse width, pulse frequency, burst duration, burst frequency, or other start/stop parameters of the stimulation.

The controller 1444 may also include a physiologic feedback module 1450. The physiologic feedback module 1450 may include a physiologic signal receiver 1451 to receive signal(s) from physiologic response sensor(s) 1452,and a physiologic parameter analyzer 1453 configured to analyze the received signal to provide feedback used to control the stimulation. The controller 1444 may also include a mapping module 1454 used to control a mapping process and store the results from the mapping.

The controller 1444 may further include a nerve block controller 1455 configured to control the nerve block stimulator 1439 and a neural stimulation controller 1456 configured to control the neural stimulator 1440. The nerve block controller 1445 and neural stimulation controller 1446 may use information from the physiologic feedback module 1450, stimulation protocol selector 1447, and mapping module 1454.

The controller 1444 may further include a timing module 1457 configured to control the timing for the blocking stimulation and the neural stimulation. By way of example, the timing information may include a schedule of start and stop times, a duration of the stimulation, coordinated timing between the blocking stimulation and neural stimulation.

The controller 1444 may further include a titration control module 1458, which may be used to adjust the nerve block and/or the neural stimulation therapy to capture the desired axons. The titration control module may be configured for adjusting at least one parameter of the neural stimulation or at least one parameter of the blocking stimulation to capture desired axons. For example, current amplitude may be adjusted, where an increase in current is expected to capture more axons. Additionally, increased pulse width may capture more axons. Increased duty cycles of neural stimulation may be used to increase a dose of neurostimulation to achieve a desired physiological effect. The titration control module may include algorithms for determining activation thresholds and saturation thresholds for neural stimulation. The titration control module may include algorithms for determining frequency thresholds depleting neurotransmitters for different types of neural tissue.

The controller 1444 may further be configured to receive an input 1459 from the clinician or patient. The input may be a start and /or stop command for a given therapy. The input may be a value of an externally-measured parameter such a blood pressure.

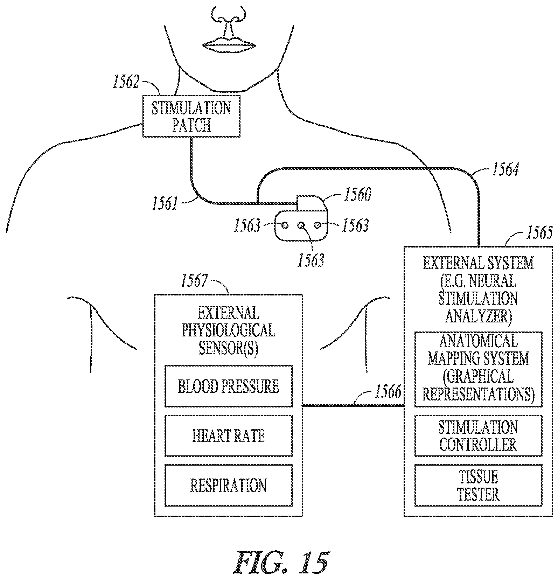

FIG. 15 illustrates, by way of example and not limitation, an implantable neural stimulator and an embodiment of an external system such as a neural stimulation system analyzer. The illustrated implantable neural stimulator 1560 may be placed subcutaneously or submuscularly in a patient's chest with a lead 1561 positioned to stimulate baroreceptor in a carotid sinus region. The lead may include a stimulation patch 1562 with a plurality of electrodes. The illustrated system provides a lead to the right carotid sinus region. The lead could be routed to the left carotid sinus region. Some embodiments may use leads to stimulate both the left and right carotid sinus regions. The neural stimulator may include leadless ECG electrodes 1563 on the housing of the device, which are capable of being used to detect heart rate, for example, to provide feedback for the neural stimulation therapy. At the time of the implantation of the neural simulator, a test lead cable 1564 may be temporarily connected to the implanted neural stimulation lead 1561 to enable a neural stimulation system analyzer 1565 to determine an appropriate placement of the lead, and verify the integrity of the stimulation path within the lead. Sensor cable(s) 1566 connect the external system 1565 to external physiology sensors 1567. These sensor(s) are used by the analyzer to detect an autonomic response to the neural stimulation. For example, heart rate, blood pressure, or respiration may be sensed to detect a baroreflex response. The external analyzer 1565 may include a controller. The external analyzer 1565 may include an anatomical mapping system configured to provide graphical representations of the anatomy based on the measured electrical characteristics of the tissue. These graphical representations may also, in some examples, be based on monitored physiological responses, such as from external physiological sensor(s) 1567 or other sensors. The graphical representations may also account for represent the cardiac cycle and/or respiratory cycle during the measurements. The anatomical mapping system may be used to virtually mark the tissue to identify a therapeutic target.

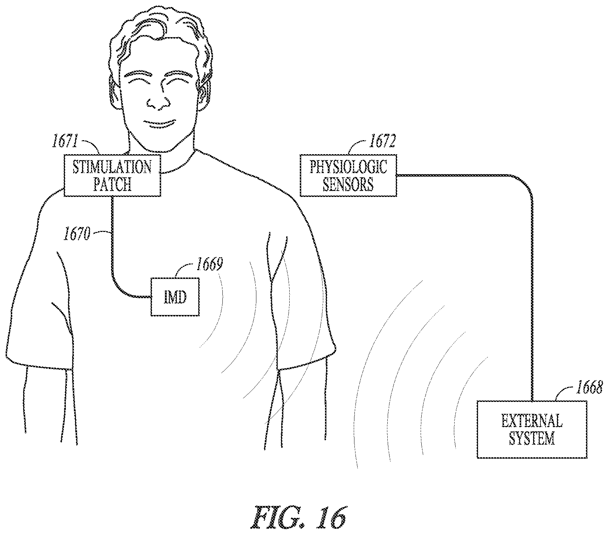

FIG. 16 illustrates, by way of example and not limitation, a system embodiment for mapping a baroreflex region which uses an external system 1668 to communicate with an implantable device 1669 to control the process to identify the carotid body and / or baroreceptor regions. The illustrated implantable device 1669 may be configured to deliver blocking stimulation to inhibit a chemoreflex response or stimulate a baroreceptor region to elicit a baroreflex response using a lead 1670 with a stimulation patch 1671. An external 1668 is configured to wirelessly communicate with the IMD 1669. One example of an external device is a programmer. The external system 1668 can be used to control the process to identify the carotid body and / or baroreceptor regions, and can use physiologic sensors 1672 to monitor the response to the stimulation.

The present subject matter may be used to stimulate desired neural pathways, and may be used to provide a depletion block on neural pathway on which it is not desired to stimulate. Examples of unwanted stimulation may include stimulation of nerves that innervate a chemoreceptor region, stimulation of nerves that conduct sensations of pain, and stimulation of nerves that innervate muscle. An example may include stimulating nerves that innervate a baroreceptor region and providing a depletion block for nerves that innervate chemoreceptors. Some embodiments may provide a depletion block for pain nerves and stimulate nerves that innervate a baroreceptor region. Some embodiments may provide a depletion block for motor nerves and stimulate nerves that innervate a baroreceptor region.

The present subject matter may be implanted for the left or right carotid sinus regions. Further, some embodiments may be configured to bilaterally stimulate the carotid sinus regions. By way of example and not limitation, both blocking stimulation and neural stimulation may be delivered to both the left and right carotid sinus regions. For example, a depletion block may be delivered to the carotid body or at least one nerve innervating the carotid body in both the right and left carotid sinus region, and a baroreceptors region or at least one nerve innervating the baroreceptors region may be stimulated in both the right and left carotid sinus regions. Some embodiments may deliver block stimulation to one of the carotid sinus regions and deliver neural stimulation to the other of the carotid sinus regions. For example, a depletion block may be delivered to the carotid body or nerve(s) innervating the carotid body in one of the right or left carotid sinus regions, and a neural stimulation may be delivered to a baroreceptors region or nerve(s) innervating the baroreceptor region in the other of the right or left carotid sinus regions.

The above detailed description is intended to be illustrative, and not restrictive. Other embodiments will be apparent to those of ordinary skill in the art upon reading and understanding the above description. As will be understood by those of ordinary skill in the hart, hardware, software and/or firmware may be used to implement various modules disclosed herein. The scope of the disclosure should, therefore, be determined with references to the appended claims, along with the full scope of equivalents to which such claims are entitled.

## Claims

1. A system, comprising:
at least one electrode for placement on tissue in a carotid sinus region;
a stimulator configured to use the at least one electrode to deliver neural stimulation to a baroreceptor region or at least one nerve innervating the baroreceptor region in the carotid sinus region to elicit a baroreflex response, and to deliver a blocking stimulation to a carotid body or at least one nerve innervating the carotid body in the carotid sinus region to inhibit a chemoreceptor response, the stimulator configured to simultaneously deliver the neural stimulation and the blocking stimulation.

2. The system of claim 1, wherein the stimulator is configured to use a stimulation vector to deliver the blocking stimulation and to use the stimulation vector to deliver the neural stimulation.

3. The system according to any of the preceding claims, wherein the at least one electrode includes an electrode for placement on or near a carotid body, and the stimulator is configured to use the electrode to both deliver the blocking stimulation and deliver the neural stimulation.

4. The system of claim 1, wherein the at least one electrode includes both a first electrode and a second electrode for placement on tissue in the carotid sinus region, and the stimulator is configured to use the first electrode to deliver neural stimulation to a baroreceptor region or a nerve innervating the baroreceptor region in the carotid sinus region to elicit a baroreflex response, and configured to use the second electrode to deliver a blocking stimulation to a carotid body or a nerve innervating the carotid body in the carotid sinus region to inhibit a chemoreceptor response, the stimulator configured to simultaneously deliver the neural stimulation and the blocking stimulation.

5. The system according to any of the preceding claims, wherein the blocking stimulation includes a synaptic junction depletion block stimulation, the depletion block stimulation including a series of pulses at a depletion pulse frequency within a range between about 100 Hz to about 1 kHz.

6. The system of claim 5, further comprising a titration control module configured to adjust a frequency of the depletion block stimulation to discriminate between depletion of one neurotransmitter from depletion of another neurotransmitter.

7. The system according to any of claims 1-4, wherein the blocking stimulation includes a kHz high frequency nerve block stimulation to prevent action potentials from propagating in axons, the kHz high frequency nerve block stimulation including a series of pulses at a frequency over 1 kHz.

8. The system according to any of the preceding claims, wherein the neural stimulation includes a series of pulses at a frequency between 0.25 Hz to 50 Hz.

9. The system according to any of the preceding claims, further comprising at least one physiologic response sensor operably connected to the controller, wherein the controller is configured to control the stimulator to apply a test stimulation to a test region within the carotid sinus region, and use the at least one physiologic response sensor to monitor a physiological response to the stimulation to determine if the test region is a carotid body or a nerve innervating the carotid body, or is a baroreceptor region or the nerve innervating the baroreceptor region.

10. The system of claim 9, wherein the at least one physiologic response sensor includes a sensor configured to sense a respiration parameter, and the controller is configured to use the sensed respiration parameter to determine if the test region is the carotid body.

11. The system according to any of claims 9-10, wherein the test stimulation to the test region includes a blocking stimulation to the carotid body.

12. The system according to any of the preceding claims, wherein the at least one physiologic response sensor includes a sensor configured to sense a blood pressure or a sensor configured to sense heart rate, and the controller is configured to use the sensed blood pressure or the sensed heart rate to determine if the test region is the baroreceptor region or the carotid sinus nerve.

13. The system of claim 12, wherein the at least one physiologic response sensor includes an accelerometer, and the system is configured to use the accelerometer to detect heart rate from blood flow through a carotid artery and/or
wherein the system is configured to use the accelerometer to sense respiration.

14. The system according to any of the preceding claims, further comprising a patch configured for implantation at the carotid sinus region, the patch to provide a substrate for the electrodes.

15. The system according to any of the preceding claims, further comprising switches, under control of the controller, to connect the stimulator to:
a nerve block selection of electrodes for use in the nerve block stimulation; or
a neural stimulation selection of electrodes for use in the neural stimulation,
wherein the stimulator includes a nerve block stimulator configured to deliver the blocking stimulation and a neural stimulator configured to deliver the neural stimulation, and the switches are under control of the controller to connect the nerve block selection of electrodes to the nerve block stimulator, and to connect the neural stimulation selection of electrodes to the neural stimulator.

## Patentansprüche

1. System, welches aufweist:
zumindest eine Elektrode zur Anordnung auf Gewebe in einem Halsschlagader-Sinusbereich;
einen Stimulator, der konfiguriert ist zur Verwendung der zumindest einen Elektrode für die Lieferung einer Nervenstimulation zu einem Barorezeptorbereich oder zumindest zu einem den Barorezeptorbereich in dem Halsschlagader-Sinusbereich versorgenden Nerv, um eine Baroreflexantwort hervorzurufen, und zum Liefern einer Blockadestimulation zu einem Halsschlagaderkörper oder zu zumindest einem den Halsschlagaderkörper in dem Halsschlagader-Sinusbereich versorgenden Nerv, um eine Chemorezeptorantwort zu unterbinden, wobei der Stimulator konfiguriert ist zum gleichzeitigen Liefern der Nervenstimulation und der Blockadestimulation.

2. System nach Anspruch 1, bei dem der Stimulator konfiguriert ist zum Verwenden eines Stimulationsvektors für die Lieferung der Blockadestimulation und zum Verwenden des Stimulationsvektors für die Lieferung der Nervenstimulation.

3. System nach einem der vorhergehenden Ansprüche, bei dem die zumindest eine Elektrode eine Elektrode zum Anordnen auf einem oder nahe eines Halsschlagaderkörpers enthält, und der Stimulator konfiguriert ist zum Verwenden der Elektrode sowohl zur Lieferung der Blockadestimulation als auch zur Lieferung der Nervenstimulation.

4. System nach Anspruch 1, bei dem die zumindest eine Elektrode sowohl eine erste Elektrode als auch eine zweite Elektrode enthält für die Anordnung auf Gewebe in dem Halsschlagader-Sinusbereich, und der Stimulator konfiguriert ist zur Verwendung der ersten Elektrode zur Lieferung von Nervenstimulation zu einem Barorezeptorbereich oder einem den Barorezeptorbereich in dem Halsschlagader-Sinusbereich versorgenden Nerv, um eine Baroreflexantwort hervorzurufen, und konfiguriert ist zum Verwenden der zweiten Elektrode für die Lieferung einer Blockadestimulation zu einem Halsschlagaderkörper oder einem den Halsschlagaderkörper in dem Halsschlagader-Sinusbereich versorgenden Nerv, um eine Chemorezeptorantwort zu unterbinden, wobei der Stimulator zum gleichzeitigen Liefern der Nervenstimulation und der Blockadestimulation konfiguriert ist.

5. System nach einem der vorhergehenden Ansprüche, bei dem die Blockadestimulation eine Synapsenverbindungs-Dezimierungsblockadestimulation enthält, wobei die Dezimierungsblockadestimulation eine Reihe von Impulsen bei einer Dezimierungsimpulsfrequenz mit einem Bereich zwischen etwa 100 Hz bis etwa 1 kHz enthält.

6. System nach Anspruch 5, weiterhin enthaltend ein Titrationssteuermodul, das konfiguriert ist zum Einstellen einer Frequenz der Dezimierungsblockadestimulation, um zwischen der Erschöpfung eines Neurotransmitters und der Erschöpfung eines anderen Neurotransmitters zu unterscheiden.

7. System nach einem der Ansprüche 1 bis 4, bei dem die Blockadestimulation eine Nervenblockadestimulation mit kHz-hoher Frequenz enthält, um zu verhindern, dass sich Aktionspotentiale in Axonen fortpflanzen, wobei die Nervenblockstimulation mit kHz-hoher Frequenz eine Reihe von Impulsen mit einer Frequenz über 1 kHz enthält.

8. System nach einem der vorhergehenden Ansprüche, bei dem die Nervenstimulation eine Reihe von Impulsen bei einer Frequenz zwischen 0,25 Hz bis 50 Hz enthält.

9. System nach einem der vorhergehenden Ansprüche, weiterhin aufweisend zumindest einen Sensor für physiologische Antworten, der betriebsmäßig mit der Steuervorrichtung verbunden ist, wobei die Steuervorrichtung konfiguriert ist zum Steuern des Stimulators für die Zuführung einer Teststimulation zu einem Testbereich innerhalb des Halsschlagader-Sinusbereichs, und zum Verwenden des zumindest einen Sensors für physiologische Antworten, um eine physiologische Antwort auf die Stimulation zu überwachen für die Bestimmung, ob der Testbereich ein Halsschlagaderkörper oder ein den Halsschlagaderkörper versorgender Nerv ist, oder ein Barorezeptorbereich oder der den Barorezeptorbereich versorgende Nerv ist.

10. System nach Anspruch 9, bei dem der zumindest eine Sensor für physiologische Antworten einen Sensor enthält, der konfiguriert ist zum Erfassen eines Atmungsparameters, und die Steuervorrichtung konfiguriert ist zum Verwenden des erfassten Atmungsparameters, um zu bestimmen, ob der Testbereich der Halsschlagaderkörper ist.

11. System nach einem der Ansprüche 9 bis 10, bei dem die Teststimulation für den Testbereich eine Blockadestimulation für den Halsschlagaderkörper enthält.

12. System nach einem der vorhergehenden Ansprüche, bei dem der mindestens eine Sensor für physiologische Antworten einen Sensor, der zum Erfassen eines Blutdrucks konfiguriert ist, oder einen Sensor, der zum Erfassen der Herzfrequenz konfiguriert ist, enthält, und die Steuervorrichtung konfiguriert ist zum Verwenden des erfassten Blutdrucks oder der erfassten Herzfrequenz, um zu bestimmen, ob der Testbereich der Barorezptorbereich oder der Halsschlagader-Sinusnerv ist.

13. System nach Anspruch 12, bei dem der zumindest eine Sensor für physiologische Antworten einen Beschleunigungsmesser enthält, und das System konfiguriert ist zur Verwendung des Beschleunigungsmessers, um die Herzfrequenz anhand der Blutströmung durch die Halsschlagader zu erfassen, und/oder bei dem das System konfiguriert ist zum Verwenden des Beschleunigungsmessers, um die Atmung zu erfassen.

14. System nach einem der vorhergehenden Ansprüche, weiterhin aufweisend einen Flicken, der konfiguriert ist zur Implantation in dem Halsschlagader-Sinusbereich, wobei der Flicken ein Substrat für die Elektroden bereitstellt.

15. System nach einem der vorhergehenden Ansprüche, weiterhin aufweisend Schalter, die von der Steuervorrichtung gesteuert werden, um den Stimulator zu verbinden mit:
einer Nervenblockadeauswahl von Elektroden zur Verwendung bei der Nervenblockadestimulation; oder
einer Nervenstimulationsauswahl von Elektroden zur Verwendung bei der Nervenstimulation, wobei der Stimulator einen Nervenblockadestimulator, der zum Liefern der Blockadestimulation konfiguriert ist, und einen Nervenstimulator, der zum Liefern der Nervenstimulation konfiguriert ist, enthält, und die Schalter von der Steuervorrichtung gesteuert werden, um die Nervenblockadeauswahl von Elektroden mit dem Nervenblockadestimulator zu verbinden und die Nervenstimulationsauswahl von Elektroden mit dem Nervenstimulator zu verbinden.

## Revendications

1. Système, comprenant :
au moins une électrode qui est destinée à être placée sur un tissu dans une région du sinus carotidien ;
un stimulateur qui est configuré de manière à utiliser l'au moins une électrode de manière à délivrer une stimulation neurale à une région de barorécepteur ou à au moins un nerf qui innerve la région de barorécepteur dans la région du sinus carotidien de manière à obtenir une réponse de baroréflexe, et de manière à délivrer une stimulation de blocage à un glomus/corpuscule carotidien ou à au moins un nerf qui innerve le corpuscule carotidien dans la région du sinus carotidien de manière à inhiber une réponse de chémorécepteur, le simulateur étant configuré de manière à délivrer de façon simultanée la stimulation neurale et la stimulation de blocage.

2. Système selon la revendication 1, dans lequel le stimulateur est configuré de manière à utiliser un vecteur de stimulation pour délivrer la stimulation de blocage et de manière à utiliser le vecteur de stimulation pour délivrer la stimulation neurale.

3. Système selon l'une quelconque des revendications précédentes, dans lequel l'au moins une électrode inclut une électrode qui est destinée à être placée sur ou à proximité d'un corpuscule carotidien, et le stimulateur est configuré de manière à utiliser l'électrode pour à la fois délivrer la stimulation de blocage et délivrer la stimulation neurale.

4. Système selon la revendication 1, dans lequel l'au moins une électrode inclut à la fois une première électrode et une seconde électrode qui est destinée à être placée sur un tissu dans la région du sinus carotidien, et le stimulateur est configuré de manière à utiliser la première électrode de manière à délivrer une stimulation neurale à une région de barorécepteur ou à un nerf qui innerve la région de barorécepteur dans la région du sinus carotidien de manière à obtenir une réponse de baroréflexe, et est configuré de manière à utiliser la seconde électrode de manière à délivrer une stimulation de blocage à un corpuscule carotidien ou à un nerf qui innerve le corpuscule carotidien dans la région du sinus carotidien de manière à inhiber une réponse de chémorécepteur, le stimulateur étant configuré de manière à délivrer de façon simultanée la stimulation neurale et la stimulation de blocage.

5. Système selon l'une quelconque des revendications précédentes, dans lequel la stimulation de blocage inclut une stimulation de blocage de déplétion de jonction synaptique, la stimulation de blocage de déplétion incluant une série d'impulsions à une fréquence d'impulsions de déplétion à l'intérieur d'une plage entre environ 100 Hz et environ 1 kHz.

6. Système selon la revendication 5, comprenant en outre un module de contrôle/commande de titration qui est configuré de manière à régler une fréquence de la stimulation de blocage de déplétion de manière à réaliser une discrimination entre une déplétion d'un neurotransmetteur et une déplétion d'un autre neurotransmetteur.

7. Système selon l'une quelconque des revendications 1 à 4, dans lequel la stimulation de blocage inclut une stimulation de blocage de nerf haute fréquence de l'ordre des kilohertz de manière à empêcher que des potentiels d'action ne se propagent dans des axones, la stimulation de blocage de nerf haute fréquence de l'ordre des kilohertz incluant une série d'impulsions à une fréquence au-delà de 1 kHz.

8. Système selon l'une quelconque des revendications précédentes, dans lequel la stimulation neurale inclut une série d'impulsions à une fréquence entre 0,25 Hz et 50 Hz.

9. Système selon l'une quelconque des revendications précédentes, comprenant en outre au moins un capteur de réponse physiologique qui est connecté de manière opérationnelle au contrôleur, dans lequel le contrôleur est configuré de manière à contrôler/commander le stimulateur de manière à appliquer une stimulation de test sur une région de test à l'intérieur de la région du sinus carotidien, et de manière à utiliser l'au moins un capteur de réponse physiologique de manière à surveiller une réponse physiologique à la stimulation de manière à déterminer si la région de test est un corpuscule carotidien ou un nerf qui innerve le corpuscule carotidien, ou est une région de barorécepteur ou le nerf qui innerve la région de barorécepteur.

10. Système selon la revendication 9, dans lequel l'au moins un capteur de réponse physiologique inclut un capteur qui est configuré de manière à détecter un paramètre de respiration, et le contrôleur est configuré de manière à utiliser le paramètre de respiration détecté de manière à déterminer si la région de test est le corpuscule carotidien.

11. Système selon l'une quelconque des revendications 9 et 10, dans lequel la stimulation de test sur la région de test inclut une simulation de blocage sur le corpuscule carotidien.

12. Système selon l'une quelconque des revendications précédentes, dans lequel l'au moins un capteur de réponse physiologique inclut un capteur qui est configuré de manière à détecter une pression sanguine ou un capteur qui est configuré de manière à détecter un battement cardiaque, et le contrôleur est configuré de manière à utiliser la pression sanguine détectée ou le rythme cardiaque détecté de manière à déterminer si la région de test est la région de barorécepteur ou le nerf du sinus carotidien.

13. Système selon la revendication 12, dans lequel l'au moins un capteur de réponse physiologique inclut un accéléromètre, et le système est configuré de manière à utiliser l'accéléromètre de manière à détecter un rythme cardiaque à partir de la circulation sanguine au travers d'une artère carotide et/ou dans lequel le système est configuré de manière à utiliser l'accéléromètre de manière à détecter une respiration.

14. Système selon l'une quelconque des revendications précédentes, comprenant en outre un patch qui est configuré de manière à être implanté au niveau de la région du sinus carotidien, le patch constituant un substrat pour les électrodes.

15. Système selon l'une quelconque des revendications précédentes, comprenant en outre des commutateurs, sous la commande du contrôleur, de manière à connecter le stimulateur sur :
une sélection d'électrodes de blocage de nerf dans le but d'une utilisation au niveau de la stimulation de blocage de nerf ; ou
une sélection d'électrodes de stimulation neurale dans le but d'une utilisation au niveau de la stimulation neurale, dans lequel le stimulateur inclut un stimulateur de blocage de nerf qui est configuré de manière à délivrer la stimulation de blocage et un stimulateur neural qui est configuré de manière à délivrer la stimulation neurale, et les commutateurs sont sous la commande du contrôleur de manière à connecter la sélection d'électrodes de blocage de nerf sur le stimulateur de blocage de nerf et de manière à connecter la sélection d'électrodes de stimulation neurale sur le stimulateur neural.
